# EUROPEAN PATENT SPECIFICATION

(11) **EP 3 289 959 B1**
(45) Date of publication and mention of the grant of the patent: **25.09.2019**
(21) Application number: 17187317.7
(22) Date of filing: 22.08.2017
(51) Int. Cl.: A61B 1/00

(54) **ENDOSCOPE**
ENDOSKOP
ENDOSCOPE

(30) Priority: 29.08.2016 JP 2016166766
(43) Date of publication of application: 07.03.2018
(73) Proprietor: Fujifilm Corporation, Minato-ku Tokyo 106-8620 (JP)
(72) Inventor: ANDO, Tadashi, Kanagawa, 258-8538 (JP)
(74) Representative: Klunker IP Patentanwälte PartG mbB

(56) References cited:
- EP-A1- 1 870 014
- EP-A1- 2 075 610
- EP-A1- 2 672 878
- US-A- 5 725 475

## Description

### BACKGROUND OF THE INVENTION

### 1. Field of the Invention

The present invention relates to an endoscope, and more particularly, to an endoscope that includes a tip portion body and a cap at a tip portion of an insertion unit.

### 2. Description of the Related Art

An endoscope disclosed in JP1998-127567A (JP-H10-127567A) includes a tip forming portion that is made of metal and is provided at a tip portion of an insertion unit, and a cap is detachably mounted on a tip surface of the tip forming portion. The cap of JP1998-127567A (JP-H10-127567A) includes a tip cover and an annular wall that are made of a resin or the like. A convex engagement portion provided on the inner surface of the annular wall is engaged with a recessed portion, which is formed on the outer peripheral surface of the tip forming portion, while being elastically deformed. As a result, the tip cover is mounted on and fixed to the outside of the tip forming portion. The tip cover is provided with an observation window through which an operator observes the inside of a body cavity, nozzles that eject washing water or blow air to the observation window to wash the observation window, and an illumination lens that illuminates the inside of the body cavity. Further, the outer peripheral surface of the tip forming portion is coated with an outer cover, and the outer cover is fixed to the tip forming portion by an adhesive portion.

An endoscope disclosed in JP2005-58400A includes a cylindrical tip portion body that is provided at the tip portion of an insertion unit, and a cap is mounted on the tip surface of the tip portion body. The cap of JP2005-58400A includes a tip cap and a flange portion. A tip portion of the tip portion body is formed as a cylindrical portion that has a diameter smaller than the diameter of a base end portion thereof, and the flange portion of the cap is fitted to the outer periphery of the cylindrical portion having a small diameter, so that the tip cap is connected to the tip portion body. The flange portion is formed in a cylindrical shape on a base end face of the tip cap so as to extend toward the tip portion body. Further, a connecting portion between the tip portion body and the tip cap is covered with angle rubber, the tip side of the angle rubber is fixed by being fastened with yarn, and an epoxy adhesive is applied to the portion of the yarn and is solidified.

### SUMMARY OF THE INVENTION

However, since the endoscope of JP1998-127567A (JP-H10-127567A) has a structure in which the annular wall is formed on the base end face of the tip cover (corresponding to the cap body) so as to extend toward the tip forming portion (corresponding to the tip portion body) and the convex engagement portion provided on the inner surface of the annular wall is engaged with the recessed portion of the outer peripheral surface of the tip forming portion, the thickness of the extending annular wall needs to be ensured. For this reason, the endoscope of JP1998-127567A (JP-H10-127567A) has a problem that the outer diameter of the tip portion of the insertion unit is increased by the thickness of the annular wall.

The endoscope of the JP2005-58400A also has this problem. That is, since the endoscope of JP2005-58400A has a structure in which the cylindrical flange portion is formed on the base end face of the tip cap (corresponding to the cap body) so as to extend toward the tip portion body and is fitted to the outer peripheral surface of the tip portion body, the thickness of the flange portion needs to be ensured. For this reason, the endoscope of JP2005-58400A also has a problem that the outer diameter of the tip portion of the insertion unit is increased by the thickness of the flange portion as in the endoscope of JP1998-127567A (JP-H10-127567A).

Document US5 725 475 discloses, see figure 17 there: An endoscope comprising: an insertion unit that has a longitudinal axis; a columnar tip portion body that is disposed on a tip side of the insertion unit and has a central axis parallel with the longitudinal axis of the insertion unit; a plate-like cap body that includes a base end face facing a tip surface of the tip portion body and being in contact with the tip surface; a mounting portion that includes a mounting surface where a part of an outer peripheral surface of the tip portion body is formed in a planar shape or a concave shape; an extending portion that extends toward the tip portion body from the base end face of the cap body and includes an outer surface and a contact surface provided closer to the central axis than the outer surface and being in contact with the mounting surface; a connecting portion that includes a first engagement portion formed on the mounting surface and a second engagement portion formed on the contact surface and connects the cap body to the tip portion body through engagement between the first engagement portion and the second engagement portion.

Document EP 1 870 014 discloses, see the figures: An endoscope with a coating member that covers the outer peripheral surface of the tip portion body and the outer surface of the extending portion in a state in which the cap body and the tip portion body are connected to each other by the connecting portion; and a stringy or belt-like fixing member that is provided on an outer peripheral surface of the coating member, is disposed at a position where the connecting portion is provided in a direction of the central axis, and fixes engagement between the first and second engagement portions.

The invention has been made in consideration of the above-mentioned circumstances, and an object of the invention is to provide an endoscope of which a tip portion of an insertion unit can be reduced in diameter.

In order to achieve the object of the invention, an endoscope according to the invention comprises: an insertion unit that has a longitudinal axis; a columnar tip portion body that is disposed on a tip side of the insertion unit and has a central axis parallel with the longitudinal axis of the insertion unit; a plate-like cap body that includes a base end face facing a tip surface of the tip portion body and being in contact with the tip surface; a mounting portion that includes a mounting surface where a part of an outer peripheral surface of the tip portion body is formed in a planar shape or a concave shape; an extending portion that extends toward the tip portion body from the base end face of the cap body and includes an outer surface and a contact surface provided closer to the central axis than the outer surface and being in contact with the mounting surface; a connecting portion that includes a first engagement portion formed on the mounting surface and a second engagement portion formed on the contact surface and connects the cap body to the tip portion body through engagement between the first engagement portion and the second engagement portion; a coating member that covers the outer peripheral surface of the tip portion body and the outer surface of the extending portion in a state in which the cap body and the tip portion body are connected to each other by the connecting portion; and a stringy or belt-like fixing member that is provided on an outer peripheral surface of the coating member, is disposed at a position where the connecting portion is provided in a direction of the central axis, and fixes engagement between the first and second engagement portions.

Unlike in the endoscope in the related art, in the endoscope of the invention, the annular wall or the cylindrical flange portion, which is caused by an increase in the diameter of the tip portion, is not formed on the cap body and the mounting portion is formed on the outer peripheral surface of the tip portion body, and the extending portion of the cap body is disposed in an empty space that is formed on the tip portion body by the formation of the mounting portion and the second engagement portion of the contact surface is engaged with the first engagement portion of the mounting surface, so that the cap body and the tip portion body are connected to each other. Accordingly, the diameter of the tip portion of the insertion unit of the endoscope can be reduced.

Further, in the endoscope of the invention, the stringy or belt-like fixing member is disposed on the outer peripheral surface of the coating member, which covers the connecting portion between the cap body and the tip portion body, to fix the engagement between the first and second engagement portions. Accordingly, the separation of the cap body from the tip portion body can be reliably prevented.

In an aspect of the invention, it is preferable that the outer surface of the extending portion is an arc-shaped surface of which a cross-section perpendicular to the central axis is formed in the shape of an arc.

According to the invention, the outer surface of the extending portion is a smooth arc-shaped surface. Therefore, the outer surface and the coating member can be made to be in close contact with each other. As a result, airtightness is improved.

In an aspect of the invention, it is preferable that the arc-shaped surface of the outer surface of the extending portion is connected to the outer peripheral surface of the tip portion body so as to be flush with the outer peripheral surface of the tip portion body in a state in which the first and second engagement portions are engaged with each other.

According to the invention, there is no difference in level between the outer surface of the extending portion and the outer peripheral surface of the tip portion body. Therefore, the outer peripheral surface of the tip portion body including the outer surface of the extending portion can be coated with the coating member with no gap. As a result, sealing performance is improved.

In an aspect of the invention, it is preferable that the first engagement portion of the tip portion body is a recessed portion, the second engagement portion of the extending portion is a protruding portion, and a locking surface, which is to be locked to a side wall surface formed on a tip side of the recessed portion, is formed on a tip side of the protruding portion in a state in which the protruding portion is engaged with the recessed portion.

According to the invention, the locking surface of the second engagement portion is locked to the side wall surface formed on the tip side of the first engagement portion in a case in which the first and second engagement portions are engaged each other. Therefore, the separation of the cap body from the tip portion body can be reliably prevented.

In an aspect of the invention, it is preferable that the outer surface of the extending portion is disposed at a position offset radially inward from an outer peripheral end of the cap body in a radial direction perpendicular to the central axis.

According to the invention, the outer size of the cap body can be reduced as much as the outer surface of the extending portion is offset. Therefore, the diameter of the tip portion of the insertion unit can be further reduced.

In an aspect of the invention, it is preferable that a distance of the offset is a distance equal to a thickness of the coating member or the sum of the thickness of the coating member and a thickness of the fixing member.

According to the invention, a difference in level in the radial direction between the outer peripheral end of the cap body and the outer peripheral surface of the coating member can be removed or suppressed. Therefore, an increase in the diameter of the tip portion, which is caused by a difference in level, can be prevented.

In an aspect of the invention, it is preferable that the outer peripheral surface of the tip portion body or the outer surface of the extending portion is a rough surface.

According to the invention, a force for fixing the coating member to the outer peripheral surface of the tip portion body, which includes the outer surface of the extending portion, is improved.

In an aspect of the invention, it is preferable that the mounting surface is formed in a planar shape and the contact surface is a flat surface.

According to the invention, the mounting surface and the contact surface can be easily worked and the contact surface is also easily assembled with the mounting surface.

In an aspect of the invention, it is preferable that a sealing agent is interposed between the outer peripheral surface of the tip portion body, which includes the outer surface of the extending portion, and the coating member.

According to the invention, airtightness and watertightness between the outer peripheral surface of the tip portion body, which includes the outer surface of the extending portion, and the coating member are improved.

In an aspect of the invention, it is preferable that the mounting portion includes a first mounting portion and a second mounting portion disposed at positions different from each other in a circumferential direction of the outer peripheral surface of the tip portion body and the extending portion includes a first extending portion and a second extending portion.

According to the invention, a force for connecting the cap body to the tip portion body is improved.

In an aspect of the invention, it is preferable that the first and second mounting portions are provided at positions, which are present on both sides of the central axis, on the outer peripheral surface of the tip portion body.

According to the invention, the cap body is connected to the tip portion body so as to pinch the tip portion body. Therefore, a force for connecting the cap body to the tip portion body is improved.

According to the invention, it is possible to provide an endoscope of which a tip portion of an insertion unit can be reduced in diameter.

### BRIEF DESCRIPTION OF THE DRAWINGS

Fig. 1 is a side view showing the entire structure of an endoscope according to a first embodiment.
Fig. 2 is a perspective view showing the structure of a tip portion of an insertion unit.
Fig. 3 is a cross-sectional view of a hard tip part.
Fig. 4 is a front view of a tip surface of a tip portion body.
Fig. 5 is a cross-sectional view of the tip portion body 48 and a cap body 54 that are connected to each other.
Fig. 6 is a perspective view of the tip portion body and the cap body that are to be assembled with each other.
Fig. 7 is a cross-sectional view of the tip portion body and the cap body.
Fig. 8 is a schematic front view of the tip portion body showing the shape of the outer surface of an extending portion.
Fig. 9 is a schematic front view of the tip portion body showing another shape of the outer surface of the extending portion.
Fig. 10 is a schematic front view of the tip portion body showing another shape of the outer surface of the extending portion.
Fig. 11 is a schematic front view of the tip portion body showing another shape of the outer surface of the extending portion.
Fig. 12 is a schematic front view of the tip portion body showing another shape of the outer surface of the extending portion.
Fig. 13 is a cross-sectional view of the hard tip part which is provided on the cap body and of which the extending portion is not offset.
Fig. 14 is a front view of a tip portion body showing a modification example of a position at which a mounting surface is formed.
Fig. 15 is a front view of a tip portion body showing a modification example of the shape of the mounting surface.

### DESCRIPTION OF THE PREFERRED EMBODIMENTS

A preferred embodiment of an endoscope according to the invention will be described in detail below with reference to accompanying drawings.

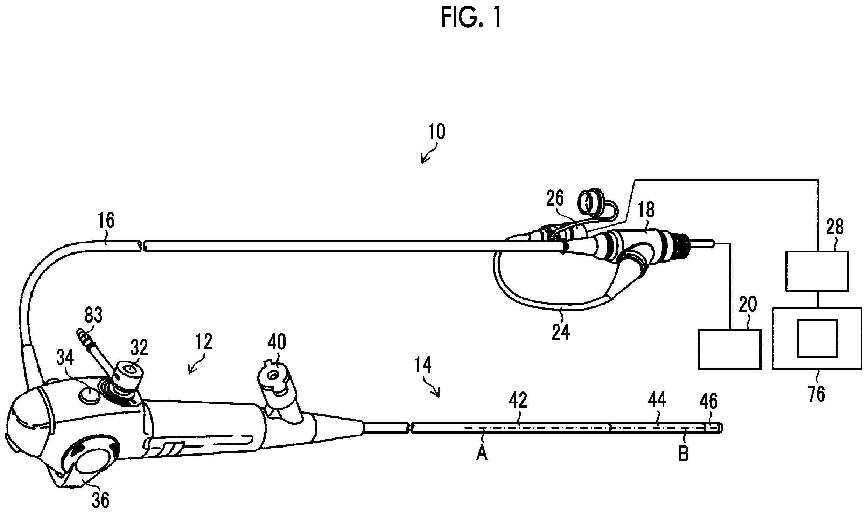

Fig. 1 is a view showing the entire structure of an endoscope 10 for a bronchial tube of an embodiment to which the endoscope of the invention is applied. The endoscope to which the invention is applied is not limited to the endoscope 10 for a bronchial tube and the invention can also be applied to other endoscopes, such as an endoscope for a large intestine.

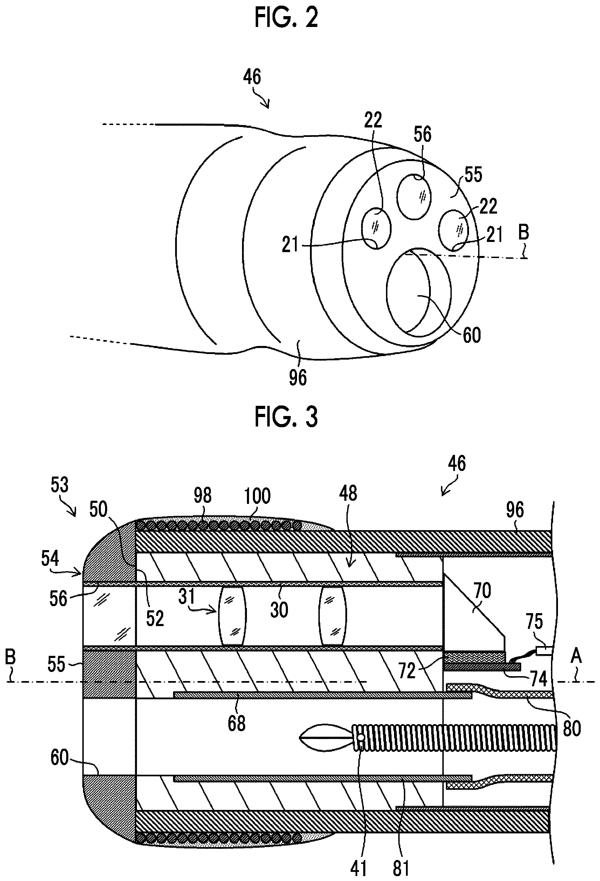

The endoscope 10 includes an operation unit 12 that is gripped by a practitioner and an insertion unit 14 of which a base end portion is connected to the operation unit 12 and which is to be inserted into a subject. A base end portion of a universal cable 16 is connected to the operation unit 12, and a light guide connector 18 is provided at a tip portion of the universal cable 16. The light guide connector 18 is connected to a light source device 20, so that illumination light is sent to illumination windows 22 and 22 (see Fig. 2) to be described below from the light source device 20. Further, an electrical connector 26 is connected to the light guide connector 18 through a cable 24, and is connected to a processor unit 28.

A suction button 32 and a shutter button 34, which are to be operated by a practitioner, are provided on the operation unit 12, and an angle lever 36 is rotatably provided on the operation unit 12. The operation unit 12 is further provided with a forceps insertion portion 40

The insertion unit 14 includes a soft part 42, a bendable part 44, and a hard tip part 46 as a tip portion that are arranged from the base end portion thereof toward the tip portion. The bendable part 44 is remotely operated so as to be bent by the rotation of the angle lever 36 of the operation unit 12. Accordingly, the hard tip part 46 can be directed in a desired direction.

Fig. 2 is an enlarged perspective view of main portions of the hard tip part 46. Fig. 3 is a cross-sectional view of the hard tip part 46 and is a cross-sectional view taken along a longitudinal axis A of the insertion unit 14 shown in Fig. 1.

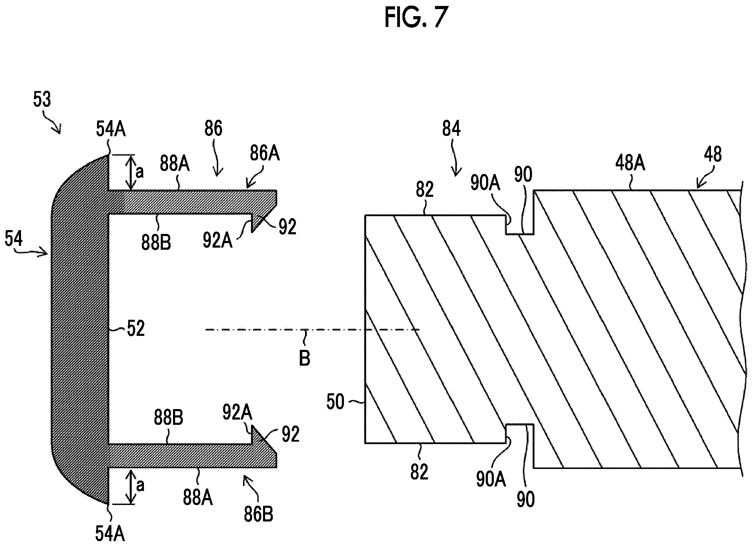

As in Fig. 3, the hard tip part 46 includes a columnar tip portion body 48 that is disposed on the tip side of the insertion unit 14 and a cap 53. Further, the cap 53 includes a disc-shaped cap body 54 including a base end face 52 that faces a tip surface 50 of the tip portion body 48 and is in contact with the tip surface 50, and extending portions 86 (see Fig. 7) to be described below. The tip portion body 48 is disposed so that a central axis B of the tip portion body 48 is parallel with the longitudinal axis A of the insertion unit 14. That is, the tip portion body 48 has the central axis B parallel with the longitudinal axis A of the insertion unit 14. A structure in which the tip portion body 48 and the cap body 54 are connected to each other will be described below.

A tip surface 55 of the cap body 54 shown in Fig. 2 is provided with a channel 56 in which a lens barrel 30 of Fig. 3 is disposed, channels 21 and 21 in which the above-mentioned illumination windows 22 and 22 are disposed, and a forceps port 60.

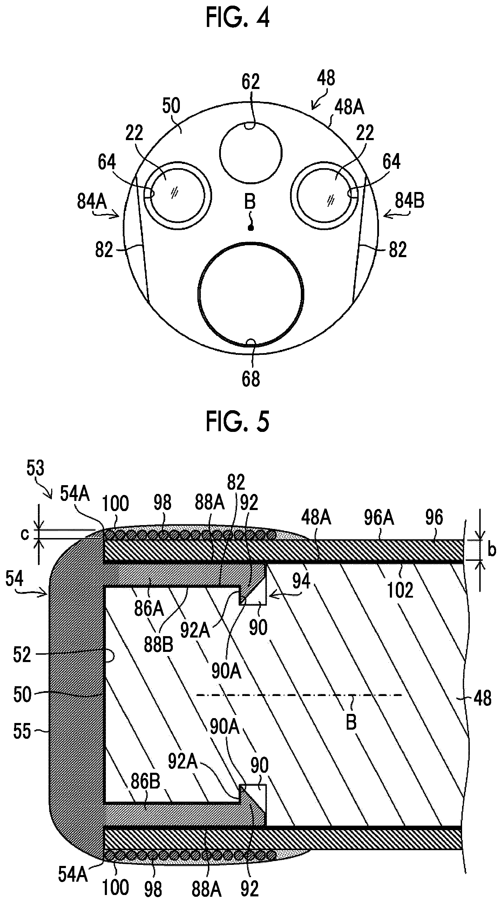

Fig. 4 is a front view showing the tip surface 50 of the tip portion body 48.

As in Figs. 3 and 4, a channel 62 in which the lens barrel 30 is disposed, channels 64 and 64 in which the illumination windows 22 and 22 and optical fibers (not shown) are disposed, and a channel 68 that faces the forceps port 60 are provided in the tip portion body 48 at predetermined positions. For example, as in Fig. 4, the channel 62 is disposed immediately above the central axis B of the tip portion body 48, the illumination windows 22 and 22 are disposed so that the channel 62 is interposed between the illumination windows 22 and 22 from the left and right, and the channel 68 is disposed immediately below the channel 62. The diameters of the channels 62, 64, and 68 are arbitrary. Further, these channels 62, 64, and 68 are provided along the central axis B of the tip portion body 48.

As in Fig. 3, the lens barrel 30 is disposed in the channel 62 and holds an observation optical system 31 including a plurality of lenses. An imaging element 72 is provided at a base end portion of the lens barrel 30 through a prism 70, and a tip portion of a signal cable 75 is connected to a substrate 74 that supports the imaging element 72. A base end portion of the signal cable 75 is inserted into the insertion unit 14, the operation unit 12, the universal cable 16, and the cable 24 of Fig. 1, extends up to the electrical connector 26, and is connected to the processor unit 28. Accordingly, an observation image, which is input from the lens barrel 30 of Fig. 3, is formed on the light-receiving surface of the imaging element 72 through the observation optical system 31 and the prism 70 disposed in the channel 62 of Fig. 3, and is converted into electrical signals by the imaging element 72. Then, the electrical signals are output to the processor unit 28 through the signal cable 75 and are converted into video signals. Accordingly, the observation image is displayed on a monitor 76 that is connected to the processor unit 28. A charge coupled device (CCD) image sensor or a complementary metal oxide semiconductor (CMOS) image sensor can be used as the imaging element 72.

Tip portions of the optical fibers (not shown) are disposed in the channels 64 and 64 of Fig. 4. Base end portions of the optical fibers are inserted into the insertion unit 14, the operation unit 12, and the universal cable 16 of Fig. 1 and extend up to the light guide connector 18. Accordingly, in a case in which the light guide connector 18 is connected to the light source device 20, illumination light emitted from the light source device 20 is transmitted to the illumination windows 22 and 22 of Fig. 2 through the optical fibers and is emitted forward from the illumination windows 22 and 22.

A tip portion of a forceps tube 80 is connected to the channel 68 of Fig. 3 through a forceps pipe 81. A base end portion of the forceps tube 80 is inserted into the insertion unit 14 of Fig. 1 and communicates with the forceps insertion portion 40. Accordingly, when various treatment tools 41, such as a forceps and a high-frequency knife, are inserted from the forceps insertion portion 40, the treatment tools 41 can be led to the outside from the forceps port 60 of Fig. 2. Further, the forceps tube 80 communicates with a suction valve (not shown) that is operated by a suction button 32 integrated with a suction connector 83 of Fig. 1. Accordingly, when a suction pump (not shown) is connected to the suction connector 83 and the suction valve is operated by the suction button 32, residues, dirt, and the like can be sucked from the forceps port 60 through the forceps tube 80.

Next, the structure in which the tip portion body 48 and the cap body 54 are connected to each other will be described below.

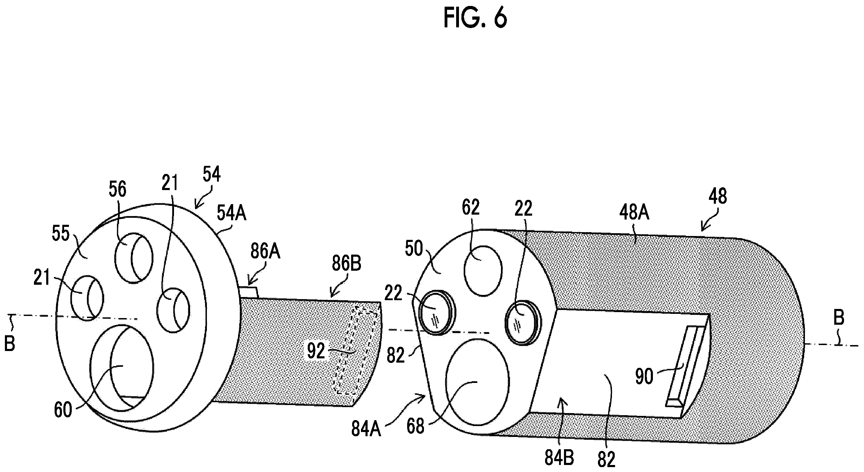

Fig. 5 is a cross-sectional view of the tip portion body 48 and the cap body 54 that are connected to each other. Fig. 6 is a perspective view of the tip portion body 48 and the cap body 54 that are to be assembled with each other. Fig. 7 is a cross-sectional view of the tip portion body 48 and the cap body 54 corresponding to Fig. 6, and is a cross-sectional view taken along the central axis B. The cross-section of the tip portion body 48 is simply shown in Figs. 5 and 7.

As in Figs. 4 to 7, the tip portion body 48 is provided with mounting portions 84 including mounting surfaces 82 that are a part, which is formed in a planar shape, of an outer peripheral surface 48A of the tip portion body 48. The mounting surface 82 is formed so that the normal direction of the mounting surface 82 corresponds to a direction orthogonal to the central axis B. However, a direction in which the mounting surface 82 is formed is not limited thereto, and the mounting surface 82 is formed so that the normal direction of the mounting surface 82 corresponds to a direction inclined with respect to the direction orthogonal to the central axis B. That is, as long as a first engagement portion 90 to be described below is formed on the base end side of the mounting surface 82, the direction in which the mounting surface 82 is formed does not matter. Further, a method of forming the mounting surface 82 is not particularly limited. For example, the outer peripheral surface 48A of the tip portion body 48 may be cut by a cutting tool to form the mounting surface 82, and the mounting surface 82 may be formed by cast molding.

It is preferable that the mounting portions 84 are provided in at least two positions in a circumferential direction of the outer peripheral surface 48A. The mounting portions 84 of the embodiment include a first mounting portion 84A and a second mounting portion 84B. Positions at which the first mounting portion 84A and the second mounting portion 84B are disposed may be positions different from each other in the circumferential direction of the outer peripheral surface 48A, and the first mounting portion 84A and the second mounting portion 84B are preferably disposed at positions, which are present on both sides of the central axis B, on the outer peripheral surface 48A of the tip portion body 48 in the embodiment. The first mounting portion 84A and the second mounting portion 84B of the embodiment are portions of the outer peripheral surface 48A, which are positioned on both sides of the channels 64 and the channel 68, and thick portions of the tip portion body 48, which do not affect the strength of the tip portion body 48, are cut out to form the mounting surfaces 82. The positions of the mounting surfaces 82 are not limited to the positions shown in Fig. 4, and it is preferable that the mounting surfaces 82 are provided at positions corresponding to the channels 62, 64, and 68, that is, positions that do not affect the strength of the tip portion body 48. The mounting portion 84 may be provided at only one position in the circumferential direction of the outer peripheral surface 48A, but it is preferable that the mounting portions 84 are provided in at least two positions as described above to stably connect the cap body 54 to the tip portion body 48.

The cap body 54 is provided with the extending portions 86 that extend toward the tip portion body 48 from the base end face 52 of the cap body 54 and include outer surfaces 88A and flat surfaces 88B as contact surfaces provided closer to the central axis B than the outer surfaces 88A and being in contact with the mounting surfaces 82. It is preferable that the extending portions 86 of the embodiment are provided in at least two positions so as to correspond to the mounting portions 84. The extending portions 86 of the embodiment include a first extending portion 86A and a second extending portion 86B. Further, the first extending portion 86A and the second extending portion 86B extend along the central axis B. The extending portion 86 may be provided at only one position, but it is preferable that the extending portions 86 are provided in at least two positions as described above to stably connect the cap body 54 to the tip portion body 48. Further, the flat surfaces 88B are formed to be flat along the mounting surfaces 82, and are in close contact with the mounting surfaces 82.

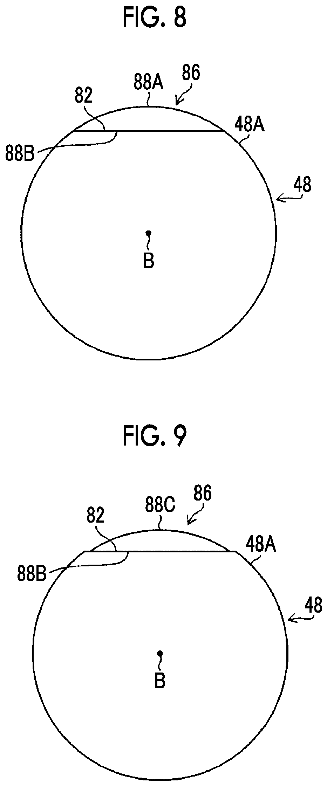

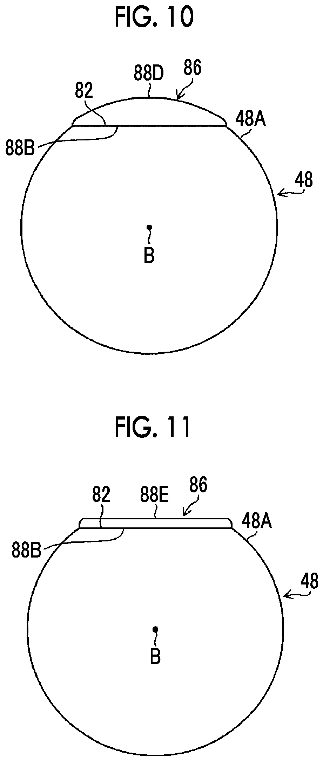

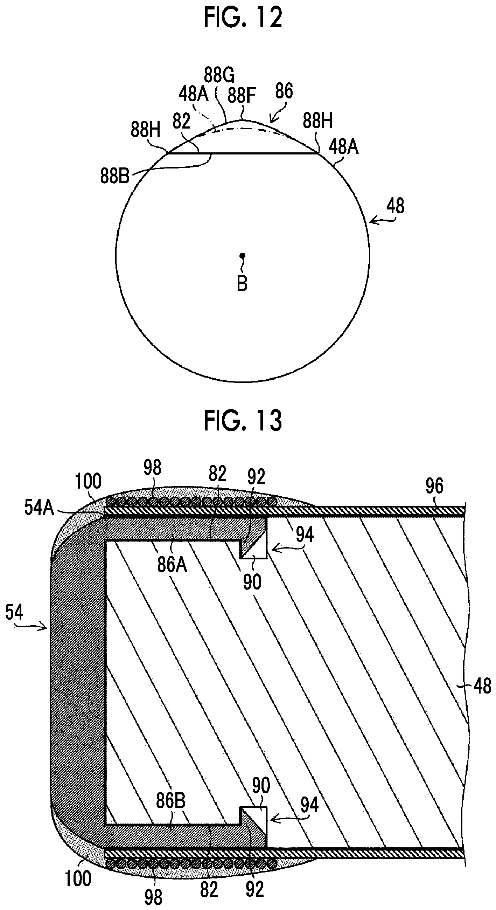

The outer surface 88A of the extending portion 86 is an arc-shaped surface of which the cross-section perpendicular to the central axis B is formed in the shape of an arc as in a schematic front view of the tip portion body 48 shown in Fig. 8. The outer surface 88A is connected to the outer peripheral surface 48A of the tip portion body 48 so as to be flush with the outer peripheral surface 48A in a state in which first engagement portions 90 and second engagement portions 92 to be described below are engaged with each other. That is, the outer surface 88A is formed of an arc-shaped surface that has a radius of curvature equal to the radius of curvature of the outer peripheral surface 48A, and both end portions of the arc-shaped surface are connected to the outer peripheral surface 48A. The arc-shaped surface of the outer surface 88A is not limited to an arc-shaped surface that is connected to the outer peripheral surface 48A so as to be flush with the outer peripheral surface 48A. For example, the outer surface of the extending portion 86 may be an outer surface 88C that is formed of an arc-shaped surface having a radius of curvature smaller than the radius of curvature of the outer peripheral surface 48A as in a modification example shown in Fig. 9, and may be an outer surface 88D that is formed of an arc-shaped surface having a radius of curvature larger than the radius of curvature of the outer peripheral surface 48A as in another modification example shown in Fig. 10. Further, the shape of the outer surface 88A is not limited to the shape of the arc-shaped surface. For example, the outer surface of the extending portion 86 may be an outer surface 88E formed of a flat surface as in a modification example shown in Fig. 11. In the case of this shape, it is preferable that the edge portions of the outer surface 88E are chamfered. Furthermore, as in another modification example shown in Fig. 12, the outer surface of the extending portion 86 may be an outer surface 88G having a shape in which a middle portion 88F protrudes radially outward from the circumference of the outer peripheral surface 48A. That is, the outer surface 88G is formed in a shape in which the outer surface is gradually separated radially outward from the circular outer peripheral surface 48A shown by a one-dot chain line in a case in which the mounting portion is not formed, as going toward the middle portion 88F from both end portions 88H. Specifically, the outer surface 88G is formed so that the radius of curvature of the arc-shaped surface of the middle portion 88F is smaller than the radius of curvature of the outer peripheral surface 48A and the radius of curvature of the surface of each of the both end portions except for the middle portion 88F is larger than the radius of curvature of the outer peripheral surface 48A.

In regard to the structure in which the tip portion body 48 and the cap body 54 are connected to each other, connecting portions 94 (see Fig. 5) including first engagement portions 90 formed on the mounting surfaces 82 and second engagement portions 92 formed on the flat surfaces 88B as in Fig. 7 are provided and the first engagement portions 90 and the second engagement portions 92 are engaged with each other, so that the cap body 54 and the tip portion body 48 are connected to each other.

In the embodiment, the first engagement portion 90 is a recessed portion and the second engagement portion 92 is a protruding portion. Further, it is preferable that a locking surface 92A, which is to be locked to a side wall surface 90A formed on the tip side of each first engagement portion 90, is formed on the tip side of each second engagement portion 92 in a state in which the second engagement portions 92 are engaged with the first engagement portions 90. The first engagement portion 90 may be a protruding portion and the second engagement portion 92 may be a recessed portion, and the first engagement portion 90 may include a locking surface and the second engagement portion 92 may include a side wall surface.

It is preferable that the outer surfaces 88A of the first and second extending portions 86A and 86B are disposed at positions that are offset radially inward from an outer peripheral end 54A of the cap body 54 in a radial direction perpendicular to the central axis B.

Further, the endoscope includes a coating member 96 that covers the outer peripheral surface 48A of the tip portion body 48 and the outer surfaces 88A of the first and second extending portions 86A and 86B in a state in which the tip portion body 48 and the cap body 54 are connected to each other by the connecting portions 94. The coating member 96 is, for example, a tube made of rubber and is elastically in close contact with the outer peripheral surface 48A and the outer surfaces 88A.

A filamentous fixing member 98 is provided on an outer peripheral surface 96A of the coating member 96. The fixing member 98 is disposed at a position where the connecting portions 94 are provided in the direction of the central axis B, and fastens and fixes engagement between the first and second engagement portions 90 and 92 through the coating member 96 in the radial direction. The fixing member 98 is fixed to the outer peripheral surface 96A by an adhesive 100 that is applied to the outer peripheral surface 96A. A stringy or belt-like fixing member may be used instead of the filamentous fixing member 98. Here, a filamentous member means that a slender and long member like yarn, and also includes a member that can be regarded as a piece of yarn as a whole by the entanglement of a plurality of fibers or the twist of a plurality of pieces of yarn, other than a piece of single yarn. Further, a stringy member means a member corresponding to the superordinate concept of a filamentous member, and includes a flexible rod-like member and also includes an elongated hollow member, such as a tube, other than a solid member. Furthermore, a belt-like member means a substantially long plate-like member that has a long length and a thickness smaller than a width as a whole. The stringy (including filamentous) or belt-like fixing member 98 is wound around the outer peripheral surface 96A of the coating member 96.

According to the endoscope 10 having the above-mentioned structure, the following effects are obtained.

According to the endoscope 10 of the embodiment, unlike in the endoscope in the related art, an annular wall or a cylindrical flange portion, which is caused by an increase in the diameter of the tip portion, is not formed on the cap body and the mounting portions 84 are formed on the outer peripheral surface 48A of the tip portion body 48. Further, the extending portions 86 of the cap body 54 are disposed in the empty spaces that are formed on the tip portion body 48 by the formation of the mounting portions 84 and the second engagement portions 92 are engaged with the first engagement portions 90, so that the tip portion body 48 and the cap body 54 are connected to each other.

Accordingly, according to the endoscope 10 of the embodiment, the spaces of the mounting portions 84 are effectively used for the connection between the tip portion body 48 and the cap body 54. Therefore, the diameter of the hard tip part 46, which is the tip portion of the insertion unit 14, can be made smaller than that of the endoscope in the related art that is provided with the annular wall or the cylindrical flange portion formed on the cap body.

Further, in the endoscope 10 of the embodiment, the stringy or belt-like fixing member 98 is disposed on the outer peripheral surface of the coating member 96, which covers the connecting portions 94 between the tip portion body 48 and the cap body 54, to fix the engagement between the first and second engagement portions 90 and 92. Accordingly, the separation of the cap body 54 from the tip portion body 48 can be reliably prevented.

Furthermore, the outer surface 88A of the extending portion 86 is an arc-shaped surface of which the cross-section perpendicular to the central axis B is formed in the shape of an arc. Accordingly, according to the endoscope 10 of the embodiment, the outer surface 88A of the extending portion 86 is a smooth arc-shaped surface. Therefore, the outer surface 88A and the coating member 96 can be made to be in close contact with each other. As a result, airtightness is improved.

Moreover, the arc-shaped surface of the outer surface 88A is connected to the outer peripheral surface 48A of the tip portion body 48 so as to be flush with the outer peripheral surface 48A in a state in which the first engagement portions 90 and the second engagement portions 92 are engaged with each other. Accordingly, according to the endoscope 10 of the embodiment, there is no difference in level between the outer surface 88A of the extending portion 86 and the outer peripheral surface 48A of the tip portion body 48. Therefore, the outer peripheral surface 48A of the tip portion body 48 including the outer surfaces 88A of the extending portions 86 can be coated with the coating member 96 with no gap. As a result, sealing performance is improved.

Further, the first engagement portion 90 is a recessed portion, the second engagement portion 92 is a protruding portion, and the locking surface 92A, which is to be locked to the side wall surface 90A formed on the tip side of each first engagement portion 90, is formed on the tip side of each second engagement portion 92 in a state in which the second engagement portions 92 are engaged with the first engagement portions 90. Accordingly, according to the endoscope 10 of the embodiment, the locking surface 92A of the second engagement portion 92 is locked to the side wall surface 90A formed on the tip side of the first engagement portion 90 in a case in which the first and second engagement portions 90 and 92 are engaged each other. Therefore, the separation of the cap body 54 from the tip portion body 48 can be reliably prevented.

Furthermore, the outer surfaces 88A of the extending portions 86 are disposed at positions that are offset radially inward from the outer peripheral end 54A of the cap body 54 in the radial direction perpendicular to the central axis B. Accordingly, according to the endoscope 10 of the embodiment, the outer size of the cap body 54 can be reduced as much as the outer surfaces 88A of the extending portions 86 are offset. Therefore, the diameter of the hard tip part 46 can be further reduced.

Moreover, an offset distance a (see Fig. 7) is set to a distance that is equal to the thickness b of the coating member 96 shown in Fig. 5 or the sum of the thickness b of the coating member 96 and the thickness c of the fixing member 98. Accordingly, according to the endoscope 10 of the embodiment, a difference in level in the radial direction between the outer peripheral end 54A of the cap body 54 and the outer peripheral surface 96A of the coating member 96 can be removed or suppressed. Therefore, an increase in the diameter of the hard tip part 46, which is caused by a difference in level, can be prevented.

The extending portions 86 can also be provided on the cap body 54 without being offset. That is, as in a cross-sectional view of the hard tip part 46 shown in Fig. 13, the extending portions 86 may be provided so as to extend from the outer peripheral end 54A of the cap body 54.

Further, as in Fig. 6, the outer peripheral surface 48A of the tip portion body 48 or the outer surfaces 88A of the extending portions 86 are worked into a rough surface. Accordingly, according to the endoscope 10 of the embodiment, frictional resistance between the outer peripheral surface 48A of the tip portion body 48, which includes the outer surfaces 88A of the extending portions 86, and the coating member 96 is increased. Therefore, a force for fixing the coating member 96 to the outer peripheral surface 48A of the tip portion body 48, which includes the outer surfaces 88A of the extending portions 86, is improved. The above-mentioned rough surface may be applied to both the outer peripheral surface 48A and the outer surfaces 88A, and may be applied to at least one of the outer peripheral surface 48A and the outer surfaces 88A. The rough surface can be applied by sandblasting or sponge blasting.

Furthermore, since the mounting surfaces 82 are formed in a planar shape as in Fig. 4 and contact surfaces, which are to be in contact with the mounting surfaces 82, are flat surfaces 88B, it is easy to assemble the cap body 54 and to also work the tip portion body 48.

Further, it is preferable that a sealing agent 102 is interposed between the outer peripheral surface 48A of the tip portion body 48, which includes the outer surfaces 88A of the extending portions 86, and the coating member 96 as in Fig. 5. Accordingly, according to the endoscope 10 of the embodiment, airtightness and watertightness between the outer peripheral surface 48A of the tip portion body 48, which includes the outer surfaces 88A of the extending portions 86, and the coating member 96 are improved.

Furthermore, as in Figs. 4 to 7, each of the mounting portions 84 includes the first mounting portion 84A and the second mounting portion 84B and each of the extending portions 86 includes the first extending portion 86A and the second extending portion 86B. Moreover, as in Fig. 4, the first mounting portion 84A and the second mounting portion 84B are provided at positions, which are present on both sides of the central axis B, on the outer peripheral surface 48A of the tip portion body 48. Accordingly, according to the endoscope 10 of the embodiment, the cap body 54 is connected to the tip portion body 48 so as to pinch the tip portion body 48 toward the inside from the outside in the radial direction. Therefore, a force for connecting the cap body 54 to the tip portion body 48 is improved.

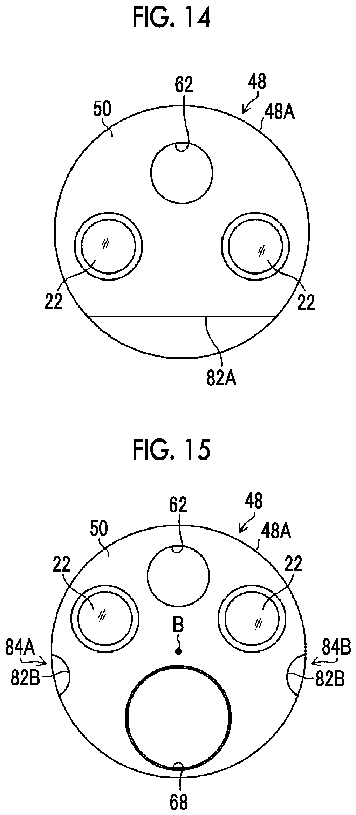

Fig. 14 is a front view of a tip portion body 48 showing a modification example of a position at which the mounting surface 82A is formed on the tip portion body 48. The tip portion body 48 shown in Fig. 14 does not include the channel 68 of the tip portion body 48 shown in Fig. 4. A part of the outer peripheral surface 48A, which is positioned below a pair of channels 64, of the tip portion body 48 of Fig. 14 is cut out in a planar shape, so that one mounting surface 82A is formed. That is, an area of the tip portion body 48, which does not affect strength, is cut out to form the mounting surface 82A.

Fig. 15 is a front view of a tip portion body 48 showing a modification example of the shapes of mounting surfaces 82B. A part of the outer peripheral surface of the tip portion body 48 is formed in a concave shape, so that the mounting surfaces 82B shown in Fig. 15 are formed. Further, the contact surfaces of extending portions 86, which are to be in contact with the concave mounting surfaces 82B, are formed as convex curved surfaces. Any shape can be applied as the shape of the mounting surface and the shape of the contact surface as long as the contact surface is in contact with the mounting surface.

### Explanation of References

- A:: longitudinal axis
- B:: central axis
- 10:: endoscope
- 12:: operation unit
- 14:: insertion unit
- 16:: universal cable
- 18:: light guide connector
- 20:: light source device
- 21:: channel
- 22:: illumination window
- 24:: cable
- 26:: electrical connector
- 28:: processor unit
- 30:: lens barrel
- 31:: observation optical system
- 32:: suction button
- 34:: shutter button
- 36:: angle lever
- 40:: forceps insertion portion
- 41:: treatment tool
- 42:: soft part
- 44:: bendable part
- 46:: hard tip part
- 48:: tip portion body
- 48A:: outer peripheral surface
- 50:: tip surface
- 52:: base end face
- 53:: cap
- 54:: cap body
- 54A:: outer peripheral end
- 55:: tip surface
- 56:: channel
- 60:: forceps port
- 62:: channel
- 64:: channel
- 68:: channel
- 70:: prism
- 72:: imaging element
- 74:: substrate
- 75:: signal cable
- 76:: monitor
- 80:: forceps tube
- 81:: forceps pipe
- 82:: mounting surface
- 82A:: mounting surface
- 82B:: mounting surface
- 83:: suction connector
- 84:: mounting portion
- 84A:: first mounting portion
- 84B:: second mounting portion
- 86:: extending portion
- 86A:: first extending portion
- 86B:: second extending portion
- 88A:: outer surface
- 88B:: flat surface
- 88C:: outer surface
- 88D:: outer surface
- 88E:: outer surface
- 88F:: middle portion
- 88G:: outer surface
- 88H:: end portion
- 90:: first engagement portion
- 90A:: side wall surface
- 92:: second engagement portion
- 92A:: locking surface
- 94:: connecting portion
- 96:: coating member
- 96A:: outer peripheral surface
- 98:: fixing member
- 100:: adhesive
- 102:: sealing agent

## Claims

1. An endoscope comprising:
an insertion unit (14) that has a longitudinal axis(A);
a columnar tip portion body (48) that is disposed on a tip side of the insertion unit (14) and has a central axis (B) parallel with the longitudinal axis (A) of the insertion unit (14);
a plate-like cap body (54) that includes a base end face (52) facing a tip surface (50) of the tip portion body (48) and being in contact with the tip surface (50);
a mounting portion (84) that includes a mounting surface (82A, B) where a part of an outer peripheral surface of the tip portion body (48) is formed in a planar shape or a concave shape;
an extending portion (86) that extends toward the tip portion body (48) from the base end face (52) of the cap body (54) and includes an outer surface (88A) and a contact surface (88B) provided closer to the central axis (B) than the outer surface and being in contact with the mounting surface (82A, B);
a connecting portion (94) that includes a first engagement portion (90) formed on the mounting surface (82A, B) and a second engagement portion (92) formed on the contact surface (86B) and connects the cap body (54) to the tip portion body (48) through engagement between the first engagement portion (90) and the second engagement portion (92);
a coating member (96) that covers the outer peripheral surface of the tip portion body (48) and the outer surface (88A) of the extending portion (86) in a state in which the cap body (54) and the tip portion body (48) are connected to each other by the connecting portion (94); and
a stringy or belt-like fixing member (98) that is provided on an outer peripheral surface of the coating member (96), is disposed at a position where the connecting portion (94) is provided in a direction of the central axis (B), and fixes engagement between the first and second engagement portions (90, 92).

2. The endoscope according to claim 1,
wherein the outer surface (88A) of the extending portion (86) is an arc-shaped surface of which a cross-section perpendicular to the central axis (B) is formed in the shape of an arc.

3. The endoscope according to claim 2,
wherein the arc-shaped surface of the outer surface (88A) of the extending portion (56) is connected to the outer peripheral surface of the tip portion body (48) so as to be flush with the outer peripheral surface of the tip portion body (48) in a state in which the first and second engagement portions (90, 92) are engaged with each other.

4. The endoscope according to claim 1, 2, or 3,
wherein the first engagement portion (90) of the tip portion body (48) is a recessed portion and the second engagement portion (92) of the extending portion is a protruding portion, and
a locking surface (92A), which is to be locked to a side wall surface formed on a tip side of the recessed portion, is formed on a tip side of the protruding portion in a state in which the protruding portion is engaged with the recessed portion.

5. The endoscope according to any one of claims 1 to 4,
wherein the outer surface (88A) of the extending portion (86) is disposed at a position that is offset radially inward from an outer peripheral end of the cap body (54) in a radial direction perpendicular to the central axis (B).

6. The endoscope according to claim 5,
wherein a distance of the offset is a distance that is equal to a thickness of the coating member (96) or the sum of the thickness of the coating member (96) and a thickness of the fixing member (98).

7. The endoscope according to any one of claims 1 to 6,
wherein the outer peripheral surface of the tip portion body (48) or the outer surface of the extending portion (86) is a rough surface.

8. The endoscope according to any one of claims 1 to 7,
wherein the mounting surface is formed in a planar shape, and the contact surface is a flat surface.

9. The endoscope according to any one of claims 1 to 8,
wherein a sealing agent (102) is interposed between the outer peripheral surface of the tip portion body (48), which includes the outer surface (88A) of the extending portion (86), and the coating member (96).

10. The endoscope according to any one of claims 1 to 9,
wherein the mounting portion (84) includes a first mounting portion (84A) and a second mounting portion (84B) that are disposed at positions different from each other in a circumferential direction of the outer peripheral surface of the tip portion body (48), and
the extending portion (86) includes a first extending portion and a second extending portion.

11. The endoscope according to claim 10,
wherein the first and second mounting portions (84A, B) are provided at positions, which are present on both sides of the central axis, on the outer peripheral surface of the tip portion body (48).

## Patentansprüche

1. Endoskop, umfassend:
eine Einführeinheit (14) mit einer Längsachse (A);
einen säulenförmigen Spitzenabschnittskörper (48) an einer Spitze der Einführeinheit (14), mit einer Mittelachse (B) parallel zu der Längsachse (A) der Einführeinheit (14);
einen plattenähnlichen Kappenkörper (54), der eine Basis-Stirnfläche (42) enthält, die zu der Spitzenfläche (50) des Spitzenabschnittskörpers (48) weist und in Berührung mit der Spitzenfläche (50) steht;
einen Halterungsabschnitt (84), der eine Halterungsfläche (82A, B) enthält, wo ein Teil der Außenumfangsfläche des Spitzenabschnittskörpers (48) zu einer ebenen oder einer konkaven Form gebildet ist;
einen Verlängerungsabschnitt (86), der sich von der Basisstimfläche (82) des Kappenkörpers (54) zu dem Spitzenabschnittskörper (48) erstreckt und eine Außenfläche (88A) und eine Kontaktfläche (88B) näher an der Mittelachse (B) als die Außenfläche und in Kontakt mit der Halterungsfläche (82A, B) enthält;
einen Verbindungsabschnitt (94), der einen ersten Eingriffsabschnitt (90) an der Halterungsfläche (82A, B) und einen zweiten Eingriffsabschnitt (92) an der Kontaktfläche (86B) enthält und den Kappenkörper (54) mit dem Spitzenabschnittskörper (48) durch Eingriff zwischen dem ersten Eingriffsabschnitt (90) und dem zweiten Eingriffsabschnitt (92) verbindet;
ein Abdeckelement (86), welches die Außenumfangsfläche des Spitzenabschnittskörpers (48) und die Außenfläche (88A) des Verlängerungsabschnitts (86) in einem Zustand abdeckt, in welchem der Kappenkörper (54) und der Spitzenabschnittskörper (48) miteinander durch den Verbindungsabschnitt (94) verbunden sind; und
ein schnurartiges oder riemenartiges Fixierelement (98), das sich an einer Außenumfangsfläche des Abdeckelements (86) befindet, an einer Stelle in einer Richtung der Mittelachse (B) angeordnet ist, wo sich der Verbindungsabschnitt (94) befindet, und den Eingriff zwischen dem ersten und dem zweiten Eingriffsabschnitt (90, 92) fixiert.

2. Endoskop nach Anspruch 1, bei dem die Außenfläche (88A) des Verlängerungsabschnitts (86) eine bogenförmige Oberfläche ist, deren Querschnitt rechtwinklig zu der Mittelachse (B) in Form eines Bogens gebildet ist.

3. Endoskop nach Anspruch 2, bei dem die bogenförmige Oberfläche der Außenfläche (88A) des Verlängerungsabschnitts (56) mit der Außenumfangsfläche des Spitzenabschnittskörpers (48) derart verbunden ist, dass sie mit der Außenumfangsfläche des Spitzenabschnittskörpers (48) in einem Zustand bündig ist, in welchem der erste und der zweite Eingriffsabschnitt (90, 92) miteinander in Eingriff stehen.

4. Endoskop nach Anspruch 1, 2 oder 3,
bei dem der erste Eingriffsabschnitt (90) des Spitzenabschnittskörpers (48) ein Vertiefungsabschnitt ist und der zweite Eingriffsabschnitt (92) des Verlängerungsabschnitts ein vorstehender Abschnitt ist, und
eine Rastfläche (92A), die mit einer Seitenwandfläche an einer Spitzenseite des vertieften Abschnitts zu verrasten ist, an einer Spitzenseite des vorstehenden Abschnitts in einem Zustand gebildet ist, in welchem der vorstehende Abschnitt mit dem vertieften Abschnitt in Eingriff steht.

5. Endoskop nach einem der Ansprüche 1 bis 4, bei dem die Außenfläche (88A) des Verlängerungsabschnitts (86) sich an einer Stelle befindet, die radial gegenüber einem Außenumfangsende des Kappenkörpers (54) in radialer Richtung rechtwinklig zu der Mittelachse (B) nach innen versetzt ist.

6. Endoskop nach Anspruch 5, bei dem ein Abstand des Versatzes ein Abstand ist, der gleich ist einer Dicke des Abdeckelements (96) oder der Summe aus der Dicke des Abdeckelements (96) und einer Dicke des Fixierelements (98).

7. Endoskop nach einem der Ansprüche 1 bis 6,
bei dem die Außenumfangsfläche des Spitzenabschnittskörpers (48) oder die Außenfläche des Verlängerungsabschnitts (86) eine raue Oberfläche ist.

8. Endoskop nach einem der Ansprüche 1 bis 7,
bei dem die Halterungsfläche eine planare Fläche ist und die Kontaktfläche eine ebene Fläche ist.

9. Endoskop nach einem der Ansprüche 1 bis 8,
bei dem ein Dichtungsmittel (102) zwischen die Außenumfangsfläche des Spitzenabschnittskörpers (48), der die Außenfläche (88A) des Verlängerungsabschnitts (86) enthält, und dem Abdeckelement (96) eingefügt ist.

10. Endoskop nach einem der Ansprüche 1 bis 9,
bei dem der Halterungsabschnitt (84) einen ersten Halterungsabschnitt (84A) und einen zweiten Halterungsabschnitt (84B) enthält, die an voneinander in Umfangsrichtung der Außenumfangsfläche des Spitzenabschnittskörpers (48) verschiedenen Stellen angeordnet sind, und
der Verlängerungsabschnitt (86) einen ersten Verlängerungsabschnitt und einen zweiten Verlängerungsabschnitt enthält.

11. Endoskop nach Anspruch 10,
bei dem der erste und der zweite Halterungsabschnitt (84A, B) an Stellen auf beiden Seiten der Mittelachse auf der Außenumfangsfläche des Spitzenabschnittskörpers (84) befinden.

## Revendications

1. Endoscope comprenant :
une unité d'introduction (14), laquelle présente un axe longitudinal (A) ;
un corps de portion de pointe colonnaire (48), lequel est disposé sur un côté de pointe de l'unité d'introduction (14) et présente un axe central (B) parallèlement à l'axe longitudinal (A) de l'unité d'introduction (14) ;
un corps de chapeau en forme de plaque (54), lequel inclut une face d'extrémité de base (52) faisant face à une surface de pointe (50) du corps de portion de pointe (48) et en contact avec la surface de pointe (50) ;
une portion de montage (84) laquelle inclut une surface de montage (82A, B) où une partie d'une surface périphérique extérieure du corps de portion de pointe (48) se présente sous une forme plane ou une forme concave ;
une portion d'extension (86), laquelle s'étend vers le corps de portion de pointe (48) à partir de la face d'extrémité de base (52) du corps de chapeau (54) et inclut une surface extérieure (88A) et une surface de contact (88B) prévue plus près de l'axe central (B) que la surface extérieure et en contact avec la surface de montage (82A, B) ;
une portion de liaison (94), laquelle inclut une première portion de prise (90) formée sur la portion de montage (82A, B) et une seconde portion de prise (92) formée sur la surface de contact (86B), et relie le corps de chapeau (54) au corps de portion de pointe (48) par le biais d'une prise entre la première portion de prise (90) et la seconde portion de prise (92) ;
un élément de revêtement (96), lequel recouvre la surface périphérique extérieure du corps de portion de pointe (48) et la surface extérieure (88A) de la portion d'extension (86) dans un état où le corps de chapeau (54) et le corps de portion de pointe (48) sont reliés l'un à l'autre par la portion de liaison (94), et
un élément de fixation de type fibres ou bande (98), lequel est prévu sur une surface périphérique extérieure de l'élément de revêtement (96), est disposé en une position où la portion de liaison (94) est prévue dans une direction de l'axe central (B), et fixe la prise entre les première et seconde portions de prise (90, 92).

2. Endoscope selon la revendication 1,
dans lequel la surface extérieure (88A) de la portion d'extension (86) est une surface de forme arquée dont la section en coupe transversale perpendiculairement à l'axe central (B) se présente sous la forme d'un arc.

3. Endoscope selon la revendication 2,
dans lequel la surface de forme arquée de la surface extérieure (88A) de la portion d'extension (56) est reliée à la surface périphérique extérieure du corps de portion de pointe (48) de manière à se trouver à ras avec la surface périphérique extérieure du corps de portion de pointe (48) dans un état où les première et seconde portions de liaison (90, 92) sont en prise l'une avec l'autre.

4. Endoscope selon la revendication 1, 2, ou 3,
dans lequel la première portion de prise (90) du corps de portion de pointe (48) est une portion en retrait, et la seconde portion de prise (92) de la portion d'extension est une portion saillante, et
une surface de verrouillage (92A), laquelle doit être verrouillée sur une surface de paroi latérale formée sur un côté de pointe de la portion en retrait, est formée sur un côté de pointe de la portion saillante dans un état où la portion saillante est en prise avec la portion en retrait.

5. Endoscope selon l'une quelconque des revendications 1 à 4,
dans lequel la surface extérieure (88A) de la portion d'extension (86) est disposée sur une position présentant un décalage de manière radiale vers l'intérieur à partir d'une extrémité périphérique extérieure du corps de chapeau (54) dans une direction radiale perpendiculaire à l'axe central (B).

6. Endoscope selon la revendication 5,
dans lequel une distance du décalage est une distance égale à une épaisseur de l'élément de revêtement (96), ou la somme de l'épaisseur de l'élément de revêtement (96) et d'une épaisseur de l'élément de fixation (98).

7. Endoscope selon l'une quelconque des revendications 1 à 6,
dans lequel la surface périphérique extérieure du corps de portion de pointe (48) ou la surface extérieure de la portion d'extension (86) est une surface rugueuse.

8. Endoscope selon l'une quelconque des revendications 1 à 7,
dans lequel la surface de montage se présente sous une forme plane, et la surface de contact est une surface plate.

9. Endoscope selon l'une quelconque des revendications 1 à 8,
dans lequel un agent d'étanchéité (102) est intercalé entre la surface périphérique extérieure du corps de portion de pointe (48), lequel inclut la surface extérieure (88A) de la portion d'extension (86), et l'élément de revêtement (96).

10. Endoscope selon l'une quelconque des revendications 1 à 9,
dans lequel la portion de montage (84) inclut une première portion de montage (84A) et une seconde portion de montage (84B), lesquelles sont disposées en des positions différentes l'une de l'autre dans une direction circonférentielle de la surface périphérique extérieure du corps de portion de pointe (48), et
la portion d'extension (86) inclut une première portion d'extension et une seconde portion d'extension.

11. Endoscope selon la revendication 10,
dans lequel les première et seconde portions de montage (84A, B) sont prévues en des positions, lesquelles sont présentes des deux côtés de l'axe central, sur la surface périphérique extérieure du corps de portion de pointe (48).
